Europäisches Patentamt

European Patent Office

Office européen des brevets

(11) Veröffentlichungsnummer: **0 293 747**
A2

# (12) EUROPÄISCHE PATENTANMELDUNG

(21) Anmeldenummer: 88108364.6

(51) Int. Cl.⁴: **C07C 51/60 , C07C 63/70**

(22) Anmeldetag: 26.05.88

(30) Priorität: 03.06.87 DE 3718524

(43) Veröffentlichungstag der Anmeldung:
07.12.88 Patentblatt 88/49

(84) Benannte Vertragsstaaten:
CH DE FR GB LI NL

(71) Anmelder: **BASF Aktiengesellschaft**
**Carl-Bosch-Strasse 38**
**D-6700 Ludwigshafen(DE)**

(72) Erfinder: **Der weitere Erfinder hat auf seine**
**Nennung verzichtet**

(54) Verfahren zur Herstellung von fluorsubstituierten Carbonsäurechloriden.

(57) Herstellung von fluorsubstituierten Carbonsäurechloriden I

$$R^F(\text{-CO-Cl})_n \qquad I$$

($R^F$ = fluorierter organischer Rest; n = 1 bis 4) aus den entsprechenden Fluoriden II

$$R^F(\text{-CO-F})_n \qquad II$$

indem man II bei erhöhter Temperatur mit Calciumchlorid umsetzt.

EP 0 293 747 A2

## Verfahren zur Herstellung von fluorsubstituierten Carbonsäurechloriden

Die vorliegende Erfindung betrifft ein neues Verfahren zur Herstellung von fluorsubstituierten Carbonsäurechloriden der allgemeinen Formel I

$$R^F(-CO-Cl)_n \qquad I$$

in welcher $R^F$ für einen ein- oder mehrfach fluorierten organischen Rest steht und n einen Wert von 1 bis 4 hat, aus den entsprechenden Säurefluoriden II

$$R^F(-CO-F)_n \qquad II$$

Verbindungen mit dem Säurerest $R^F$-CO- erhält man allgemein aus den entsprechenden Chlor- oder Bromverbindungen wie beispielsweise $R^{Cl}$-CO-OH oder $R^{Cl}$-CO-Cl, indem man das Chlor bzw. das Brom mit Fluorwasserstoff gegen Fluor austauscht (vgl. z.B. Houben-Weyl, "Methoden der organischen Chemie", Band V/3, 4. Auflage, 1962, Seiten 119-140).

Da die Säurefunktion bei dieser Austauschreaktion in der Regel am leichtesten fluoriert wird, erhält man fast immer die fluorierten Carbonsäurefluoride II, die jedoch für weitere Umsetzungen häufig schlecht geeignet sind. Dies gilt besonders für Friedel-Crafts-Acylierungen mit Aluminiumchlorid und vor allem für die Rosenmund-Reaktion, nach welcher man die Säurehalogenidgruppe mit Wasserstoff katalytisch zur Formylgruppe reduziert.

Es ist daher erforderlich, das reaktionsträge Fluor der Säurefunktion wieder durch Chlor auszutauschen, wobei indes nach den üblichen Chlorierungsmethoden auch die Rücksubstitution des im Rest $R^F$ befindlichen Fluors durch Chlor stattfindet.

Zur Vermeidung dieser unerwünschten Nebenreaktion hat man II zunächst zur Säure $R^F$-COOH hydrolysiert und die Säure sodann mit einem milden Chlorierungsmittel wie Thionylchlorid wieder chloriert ("Chemie und Technologie aliphatischer fluororganischer Verbindungen", Enke Verlag, 1964, S. 125), jedoch ist dieses Verfahren offensichtlich umständlich und unwirtschaftlich.

Auch das Verfahren der DE-A 3 129 274, nach welchem man die fluorsubstituierten Carbonsäurefluoride mit Silicium- oder Titantetrachlorid in die entsprechenden Chloride überführt, ist unbefriedigend, weil diese Reagenzien relativ teuer sind und weil die Beseitigung der hierbei entstehenden flüchtigen Silicium- und Titanfluoride Probleme aufwirft.

Der Erfindung lag daher die Aufgabe zugrunde, die fluorsubstituierten Carbonsäurechloride I aus den entsprechenden Fluoriden II auf einfachere und wirtschaftlichere Weise zugänglich zu machen als bisher.

Demgemäß wurde ein Verfahren zur Herstellung von fluorsubstituierten Carbonsäurechloriden der allgemeinen Formel I

$$R^F(-CO-Cl)_n \qquad I$$

in welcher $R^F$ für einen einfach oder mehrfach fluorierten organischen Rest steht und n einen Wert von 1 bis 4 hat, aus den entsprechenden Säurefluoriden II

$$R^F-(CO-F)_n \qquad II$$

gefunden, welches dadurch gekennzeichnet ist, daß man II bei erhöhter Temperatur mit Calciumchlorid umsetzt.

Um einen möglichst vollständigen Umsatz zu erzielen, ist es zweckmäßig, auf 1 mol II mindestens n mol Calciumchlorid einzusetzen. Die bevorzugten Mengen Calciumchlorid liegen zwischen n und 1,5 n mol, und größere Mengen als 2 n mol wirken sich im allgemeinen nicht mehr nennenswert auf die Umsetzung aus.

Im übrigen verläuft die Reaktion umso schneller, je feinteiliger das Calciumchlorid ist und je intensiver man das Reaktionsgemisch rührt.

Die Reaktion setzt in der Regel bei 50 °C ein und verläuft zwischen 80 und 120 °C mit merklicher bis wirtschaftlich zufriedenstellender Geschwindigkeit. Oberhalb von 150 °C ist mit der Substitution des im Rest $R^F$ gebundenen Flours durch Chlor als Nebenreaktion zu rechnen.

Die Reaktion ist nicht druckabhängig, weshalb man sie in der Regel bei Normaldruck ausführt, sofern das Reaktionsgemisch keine Komponenten enthält, welche bei der Reaktionstemperatur flüchtig sind. In diesen Fällen arbeitet man im geschlossenen Gefäß unter dem Eigendruck der Komponenten.

Die Mitverwendung eines Verdünnungsmittels ist nicht erforderlich, empfiehlt sich aber im allgemeinen. Als Verdünnungsmittel eignen sich inerte aprotische polare organische Flüssigkeiten, in denen sich beide Säurehalogenide I und II lösen, also beispielsweise Ether wie Dioxan, Tetrahydrofuran und die Dimethylether des Ethylenglycols, Diethylenglycols und Triethylenglycols, Amide wie Dimethylformamid und Acetamid, Lactame wie N-Methylpyrrolidon, Nitrile wie Acetonitril und Benzonitril sowie Sulfone wie Dimethylsulfon und Tetramethylensulfon. Die Menge des Verdünnungsmittels ist nicht kritisch, beträgt im allgemeinen aber nicht mehr als das 20fache der Menge von II. Vorzugsweise arbeitet man mit 5 bis 10 kg des Verdünnungsmittels pro kg II. Im Hinblick auf die Reaktionsfähigkeit der Säurehalogenide I und II sollte die Anwesenheit von protischen Verbindungen, insbesondere von Wasser, nach Möglichkeit ausgeschlossen werden.

Man kann die Reaktion nach allen Techniken vornehmen, nach denen man Feststoffe mit Flüssigkeiten umsetzt. Die einfachste und meistens voll befriedigende Ausführungsform besteht darin, eine Mischung aus dem Carbonsäurefluorid II, dem Calciumchlorid und gegebenenfalls dem Verdünnungsmittel solange unter Rühren zu erhitzen, bis sich praktisch kein Fluorid II mehr nachweisen läßt, wie durch Dünnschichtchromatographie verfolgt werden kann.

Die Aufarbeitung des Reaktionsgemisches kann wie üblich vorgenommen werden, indem man das Calciumsalz abfiltriert und das Filtrat destillativ fraktioniert.

Das gute Gelingen des erfindungsgemäßen Verfahrens ist von der Art der Ausgangsverbindung II nicht erkennbar abhängig, so daß es sich prinzipiell für beliebige fluorsubstituierte Säurefluoride als Ausgangsstoffen eignet. Verbindungen mit fluorierten Acylresten $R^F$-CO- haben vor allem auf dem Gebiet der Pflanzenschutzmittel, der Arzneimittel und der Farbstoffe Bedeutung, so daß sich die gewerbliche Anwendung des erfindungsgemäßen Verfahrens nach derartigen Endprodukten richtet. Hierbei können die Reste $R^F$ nicht nur ein oder mehrere Fluoratome sondern auch ein oder mehrere weitere inerte Substituenten wie Chlor, Brom, Nitrogruppen, Cyangruppen und tertiäre Aminogruppen enthalten, und außerdem können die Reste durch Sauerstoff und Schwefel sowie durch die Gruppierungen -CO-, -O-CO- und -SO₂- unterbrochen sein.

Als Beispiele für die in Betracht kommenden Verbindungsklassen II sowie für einzelne Vertreter hieraus seien genannt:

- fluorsubstituierte aliphatische Carbonsäurefluoride, darunter vor allem solche, in denen $R^F$ sich von $C_1$-$C_{18}$-, speziell $C_1$-$C_{12}$- und besonders $C_1$-$C_6$-Alkylresten ableitet, wobei die Alkylreste neben Fluor noch Chlor, die Trifluormethoxy- und die Trifluormethylthiogruppe tragen, also beispielsweise
Trifluoressigsäurefluorid,
4-Fluorbuttersäurefluorid,
4,4,4-Trifluorbuttersäurefluorid,
Perfluorbuttersäurefluorid,
Perfluorcaprylsäurefluorid,
Difluorchloressigsäurefluorid,
Fluordichloressigsäurefluorid,
Trifluormethoxy-difluoressigsäurefluorid;
- fluorsubstituierte cycloaliphatische Carbonsäurefluoride wie Perfluorcyclohexylcarbonsäurefluorid;
- fluorsubstituierte isocyclische und heterocyclische aromatische Carbonsäurefluoride, darunter vor allem solche, die sich von der Benzoesäure ableiten und neben Fluor noch ein oder zwei weitere Substituenten tragen, beispielsweise
2-Fluorbenzoylfluorid,
3-Fluorbenzoylfluorid,
4-Fluorbenzoylfluorid,
2-Fluor-3-chlorbenzoylfluorid,
3-Brom-4-fluorbenzoylfluorid,
3-Phenoxy-4-fluorbenzoylfluorid,
3-Fluor-4-methoxybenzoylfluorid,
2,4-Difluorbenzoylfluorid,
3,4-Difluorbenzoylfluorid,
2,5-Difluorbenzoylfluorid,
4-(4-Fluorphenyl)-benzoylfluorid,
4-Fluorpyridin-3-carbonsäurefluorid,
6-Fluorpyrazin-2-carbonsäurefluorid,
Fluortriazin(1,3,5)-carbonsäurefluorid,

4-Fluorchinolin-3-carbonsäurefluorid,

- fluorsubstituierte gemischt aliphatisch-aromatische Carbonsäurefluoride, insbesondere solche mit dem Strukturelement des Benzols und aliphatischen $C_1$-$C_6$-Resten in der Seitenkette, beispielsweise
2-Trifluormethylbenzoylfluorid,
3-Trifluormethylbenzoylfluorid,
4-Trifluormethylbenzoylfluorid,
4-Chlordifluormethylbenzoylfluorid,
2-Fluor-4-trifluormethylbenzoylfluorid,
4-Trifluormethoxybenzoylfluorid,
4-Chlordifluormethoxybenzoylfluorid,
3-Chlor-4-trifluormethylbenzoylfluorid,
4-Phenylsulfonyl-3-trifluormethylbenzoylfluorid,
α-Fluorphenylessigsäurefluorid,
3-Fluor-4-methylbenzoylfluorid,
4-Fluorbenzoylessigsäurefluorid,
4-Fluorphenoxyessigsäurefluorid,
2-Fluorphenylessigsäurefluorid,
(4-Fluor)-2-phenylacrylsäurefluorid.

Außer den Fluoriden einbasischer Carbonsäuren kommen selbstverständlich auch die Fluoride mehrbasischer Carbonsäuren als Ausgangsmaterialien für das erfindungsgemäße Verfahren in Betracht, wobei Verbindungen mit zwei bis vier, vor allem mit zwei Säurefunktionen von Interesse sind. Als derartige Ausgangsstoffe eignen sich beispielsweise
Fluorbernsteinsäuredifluorid,
Perfluorbernsteinsäuredifluorid,
Fluorterephthalsäuredifluorid,
Trifluormethylterephthalsäuredifluorid.

Die genannten fluorsubstituierten Carbonsäurefluoride sind bekannt, z.B. aus der DE-A 31 29 274, oder nach bekannten Methoden erhältlich.

Die nach dem erfindungsgemäßen Verfahren auf einfache und wirtschaftliche Weise zugänglichen fluorsubstituierten Carbonsäurefluoride I sind wertvolle Zwischenprodukte für die Synthese fluororganischer Verbindungen, wie sie vor allem auf den Gebieten der Pflanzenschutzmittel, der Arzneimittel und der Farbstoffe Bedeutung haben.

Beispiele 1 bis 6

Herstellung verschiedener fluorsubstituierter Benzoylchloride I

Eine Mischung aus a g (= a′ mol) eines fluorsubstituierten Benzoylfluorids II mit dem Substituenten $R^F$ und b g (= b′ mol) fein gepulvertes, bei 140°C getrocknetes Calciumchlorid wurde t Stunden lang bei 100°C gerührt, wonach, das Reaktionsgemisch in Methylenchlorid aufgenommen, vom Feststoff und vom Lösungsmittel befreit und destillativ auf das Benzoylchlorid aufgearbeitet wurde.

Einzelheiten und Ergebnisse dieser Versuche sind der nachstehenden Tabelle zu entnehmen.

4

| Bsp. | fluorsubst. Benzoylfluorid II | | | CaCl₂ | | | Ausbeute an |
|------|------|------|------|------|------|------|------|
| | $R^F$ | a | a' | b | b' | t | Chlorid I |
| | | [g] | [mol] | [g] | [mol] | [h] | [%] |
| 1 | 4-Trifluormethyl | 2244 | 11,7 | 1287 | 11,7 | 14 | 84 |
| 2 | 3-Trifluormethyl | 192 | 1,0 | 122 | 1,1 | 13 | 89 |
| 3 | 4-Fluor | 142 | 1,0 | 122 | 1,1 | 14 | 91 |
| 4 | 4-Chlordifluormethyl | 105 | 0,5 | 61 | 1,1 | 12 | 87 |
| 5 | 4-Chlordifluormethoxy | 105 | 0,5 | 61 | 1,1 | 12 | 87 |
| 6 | 3-Trifluormethoxy | 208 | 1,0 | 111 | 1,0 | 14 | 88 |

**Ansprüche**

Verfahren zur Herstellung von fluorsubstituierten Carbonsäurechloriden der allgemeinen Formel I

$$R^F(-CO-Cl)_n \quad I$$

in welcher $R^F$ für einen einfach oder mehrfach fluorierten organischen Rest steht und n einen Wert von 1 bis 4 hat, aus den entsprechenden Säurefluoriden II

$$R^F(-CO-F)_n \quad II$$

dadurch gekennzeichnet, daß man II bei erhöhter Temperatur mit Calciumchlorid umsetzt.